# EUROPEAN PATENT APPLICATION

(11) **EP 4 253 408 A1**
(43) Date of publication of application: **04.10.2023**
(21) Application number: 21896976.4
(22) Date of filing: 23.11.2021
(51) Int. Cl.: C07K 14/725, C12N 15/12, C12N 15/63, C12N 5/0783, C12N 5/10, A61K 35/17, A61K 38/17, A61P 35/00

(54) **TCR CAPABLE OF RECOGNIZING HPV ANTIGEN**

(30) Priority: 26.11.2020 CN 202011349425
(71) Applicant: XLifeSc, Ltd., Zhuhai, Guangdong 519031 (CN)
(72) Inventor: LI, Yi, Zhuhai, Guangdong 519031 (CN); YANG, Dongxue, Zhuhai, Guangdong 519031 (CN); CHEN, Shaopei, Zhuhai, Guangdong 519031 (CN); SUN, Hanli, Zhuhai, Guangdong 519031 (CN)
(74) Representative: J A Kemp LLP
(86) International application number: PCT/CN2021/132461
(87) International publication number: WO 2022/111475

(57) **Abstract**

The present invention provides a T cell receptor (TCR) capable of specifically binding to HPV16 E6 antigen short peptide complex TIHDIILECV-HLAA0201. Moreover, an effector cell transducing the TCR of the present disclosure also has a strong killing function. Such TCR can be used separately or in combination with other therapeutic agents, and can also be used in adoptive cellular immunotherapy to target a tumor cell presenting the complex TIHDIILECV-HLAA0201.

## Description

### TECHNICAL FIELD

The present disclosure relates to the field of biotechnology, in particular to a T cell receptor (TCR) capable of recognizing a polypeptide derived from HPV16 E6 protein, and preparation and use thereof.

### BACKGROUND

There are only two types of molecules that are capable of recognizing antigens in a specific way, one of which is immunoglobulin or antibody; and the other is the T cell receptor (TCR). TCR is a glycoprotein on the surface of the cell membrane in the form of heterodimers of alpha chain/beta chain or gamma chain/beta chain. In humans, 95% TCR of T cells are composed of alpha and beta chains encoded by TRA and TRB, respectively. Recognition of pMHC (antigen peptide-major histocompatibility complex) involves two kinds of binding: binding of TCR to MHC molecules and binding of TCR to polypeptide antigens. The binding surfaces on the TCR are from six regions: CDR1, CDR2, and CDR3 of each of the TCR alpha and beta chains. CDR1 and CDR2 mainly bind to the relatively conserved MHC molecule, while CDR3 mainly binds to the variable antigen polypeptides. This mode of binding provides a good guarantee for the specific binding of TCR to MHC molecules and enables the recognition of variable antigens. (Chinese journal of cell biology 2011, 33(9):955-963). Among them, CDR3 varies most greatly, which directly determines the antigen-binding specificity of TCR. In the immune system, direct physical contact between T cells and antigen-presenting cells (APC) is induced by the binding of antigen-specific TCR to the pMHC complex. Then the interaction of other cell membrane surface molecules of the T cells and the APCs occurs, leading to a series of subsequent cell signaling and other physiological responses. Hence, the T cells specific to different antigen exert immune effects on their target cells.

E6 gene is one of the early region genes of the human papillomavirus (HPV) genome, located at positions 83 to 559 of the HPV genome, and encodes the E6 protein. The most common type of cervical cancer worldwide is caused by HPV16, accounting for 50% to 60% of the detected cases (Acta Acad Med Sin, 2007, 29(5):678-684). The E6 protein is one of the two essential oncoproteins encoded upon high-risk HPV infection of cervical epithelial cells ([J] Journal of Jiangsu University (Medical Edition), 2018, 28(2):135-139). HPV16 E6 also causes head and neck tumors (China Journal of Otolaryngology and Craniocerebral Base Surgery, 2017, 23 (6):594-598), conjunctival intraepithelial neoplasia (CIN), and keratoconjunctival invasive squamous cell carcinoma (SCC) and other diseases. TIHDIILECV is an epitope peptide of HPV16 E6 protein and a target for the treatment of HPV16 E6-related diseases.

The present disclosure is directed to the development of TCRs that are capable of binding TIHDIILECV-HLAA0201 complexes and have high application values for treating tumors. For example, a TCR capable of targeting the tumor cell marker can be used to deliver cytotoxic agents or immunostimulants to target cells, or to be transformed into T cells such that the T cells expressing the TCR are capable of destroying the tumor cells, and thereby administered to the patient during a treatment referred to as adoptive immunotherapy.

### SUMMARY

It is an object of the present disclosure to provide a TCR that specifically recognizes and binds to the TIHDIILECV-HLAA0201 complex. It is a further object of the present disclosure to provide a method for preparing the above type of TCR and use of the above type of TCR.

In a first aspect of the present disclosure, there is provided a TCR having an activity to bind to TIHDIILECV-HLA A0201 complex. The TCR has an alpha chain variable domain having an amino acid sequence having at least 90% sequence homology to the amino acid sequence as set forth in SEQ ID NO: 1, and a beta chain variable domain having an amino acid sequence having at least 90% sequence homology to the amino acid sequence as set forth in SEQ ID NO: 5.

In another preferred embodiment, the alpha chain variable domain of the TCR includes an amino acid sequence having at least 91%, 92%, 93%, 94%, 95%, 96%, 97%, 98%, or 99% sequence homology to the sequence as set forth in SEQ ID NO: 1.

In another preferred embodiment, the beta chain variable domain of the TCR has an amino acid sequence having at least 91%, 92%, 93%, 94%, 95%, 96%, 97%, 98%, 99%, or 100% sequence homology to the sequence as set forth in SEQ ID NO: 5.

In another preferred embodiment, the TCR in the present disclosure is of human origin.

In another preferred embodiment, the TCR is an αβ heterodimeric TCR, preferably having an alpha chain constant region sequence TRAC*01 and a beta chain constant region sequence TRBC1*01 or TRBC2*01.

In another preferred embodiment, the alpha and beta chain constant regions are alpha and beta chain constant regions of mouse origin, respectively.

In another preferred embodiment, the TCR is soluble.

In another preferred embodiment, the alpha chain variable domain of the TCR includes three CDRs, and the beta chain variable domain of the TCR includes three CDRs, wherein the three CDRs of the beta chain variable domain of the TCR have amino acid sequences of:
β CDR1- MNHNS (SEQ ID NO: 17),
β CDR2- SASEGT (SEQ ID NO:18), and
β CDR3- ASGPWGSSGNTIY (SEQ ID NO:19), respectively.

In another preferred embodiment, the alpha chain variable domain of the TCR has an amino acid sequence having at least 95% sequence homology to the amino acid sequence as set forth in SEQ ID NO: 1; and the beta chain variable domain of the TCR has an amino acid sequence having at least 95% sequence homology to the amino acid sequence as set forth in SEQ ID NO: 5.

In another preferred embodiment, the amino acid sequence of the beta chain variable domain of the TCR is as set forth in SEQ ID NO: 5.

In another preferred embodiment, the alpha chain variable domain of the TCR has an amino acid sequence having at least 95% sequence homology to the amino acid sequence as set forth in SEQ ID NO: 1; and the three CDRs of the beta chain variable domain of the TCR have amino acid sequences of:
β CDR1- MNHNS (SEQ ID NO: 17),
β CDR2- SASEGT (SEQ ID NO:18), and
β CDR3- ASGPWGSSGNTI (SEQ ID NO:19), respectively.

In another preferred embodiment, the alpha chain variable domain of the TCR has an amino acid sequence having at least 95% sequence homology to the amino acid sequence as set forth in SEQ ID NO: 1; and the beta chain variable domain of the TCR has an amino acid sequence as set forth in SEQ ID NO: 5.

In another preferred embodiment, the alpha chain variable domain of the TCR includes three CDRs, and the beta chain variable domain of the TCR includes three CDRs, wherein in the alpha chain variable domain of the TCR, CDR3α has an amino acid sequence selected from the group consisting of ALRAGANNLF (SEQ ID NO:22), ALRAGANLPV (SEQ ID NO:23), ALRAGANTPI (SEQ ID NO:24), or ALRAGAWPMK (SEQ ID NO:25).

In another preferred embodiment, the alpha chain variable domain of the TCR includes three CDRs, and the beta chain variable domain of the TCR includes three CDRs, wherein in the alpha chain variable domain of the TCR, CDR1αhas an amino acid sequence of TRDTTYY; CDR2a has an amino acid sequence of RNSFDEQN; and CDR3α has an amino acid selected from the group consisting of ALRAGANNLF (SEQ ID NO:22), ALRAGANLPV (SEQ ID NO:23), ALRAGANTPI (SEQ ID NO:24), or ALRAGAWPMK (SEQ ID NO:25).

In another preferred embodiment, the three CDRs of the alpha chain variable domain of the TCR have the following sequences:
CDR1α: TRDTTYY (SEQ ID NO:20),
CDR2α: RNSFDEQN (SEQ ID NO:21), and
CDR3α: ALRAGANNLF (SEQ ID NO: 22), respectively, and
CDR3α includes at least one of the following mutations:

| Residue before mutation | Residue after mutation |
|---|---|
| N at position 7 of CDR3α | W |
| N at position 8 of CDR3α | L or T or P |
| L at position 9 of CDR3α | P or M |
| F at position 10 of CDR3α | V or I or K |

In another preferred embodiment, the three CDRs of the alpha chain variable domain of the TCR have the following sequences:
CDR1α: TRDTTYY (SEQ ID NO:20)
CDR2α: RNSFDEQN (SEQ ID NO:21), and
CDR3α: ALRAGANNLF (SEQ ID NO: 22),
CDR3α includes at least one of the following mutations:

| Residue before mutation | Residue after mutation |
|---|---|
| N at position 7 of CDR3α | W |
| N at position 8 of CDR3α | L or T or P |
| L at position 9 of CDR3α | P or M |
| F at position 10 of CDR3α | V or I or K |

and the three CDRs of the beta chain variable domain of the TCR have amino acid sequences of:
β CDR1- MNHNS (SEQ ID NO: 17),
β CDR2- SASEGT (SEQ ID NO:18), and
β CDR3- ASGPWGSSGNTIY (SEQ ID NO:19), respectively.

In another preferred embodiment, the TCR has CDRs selected from the group consisting of:

| CDR No. | CDR1α | CDR2α | CDR3α | CDR1β | CDR2β | CDR3β |
|---|---|---|---|---|---|---|
| 1 | TRDTTYY | RNSFDEQN | ALRAGANNLF | MNHNS | SASEGT | ASGPWGSSGNTIY |
| 2 | TRDTTYY | RNSFDEQN | ALRAGANLPV | MNHNS | SASEGT | ASGPWGSSGNTIY |
| 3 | TRDTTYY | RNSFDEQN | ALRAGANTPI | MNHNS | SASEGT | ASGPWGSSGNTIY |
| 4 | TRDTTYY | RNSFDEQN | ALRAGAWPMK | MNHNS | SASEGT | ASGPWGSSGNTIY |

In another preferred embodiment, the alpha chain variable domain of TCR has an amino acid sequence of any one of SEQ ID NOs: 1 to 4; and/or the beta chain variable domain of TCR has an amino acid sequence of SEQ ID NO:5.

In another preferred embodiment, the TCR is selected from the group consisting of:

| TCR No. | Sequence of alpha chain variable domain SEQ ID NO: | Sequence of beta chain variable domain SEQ ID NO: |
|---|---|---|
| 1 | 1 | 5 |
| 2 | 2 | 5 |
| 3 | 3 | 5 |
| 4 | 4 | 5 |

In another preferred embodiment, the TCR includes (i) all or part of an alpha chain of the TCR other than its transmembrane domain, and (ii) all or part of a beta chain of the TCR other than its transmembrane domain, wherein (i) and (ii) each include a variable domain chain and at least part of a constant domain of the TCR.

In another preferred embodiment, an artificial interchain disulfide bond is included between the alpha chain constant region and the beta chain constant region of the TCR.

In another preferred embodiment, a cysteine residue forming the artificial interchain disulfide bond is substituted for one or more sets of sites selected from the group consisting of:
Thr48 of TRAC^{∗}01 exon 1 and Ser57 of TRBC1^{∗}01 or TRBC2^{∗}01 exon 1;
Thr45 of TRAC^{∗}01 exon 1 and Ser77 of TRBC1^{∗}01 or TRBC2^{∗}01 exon 1;
Tyr10 of TRAC^{∗}01 exon 1 and Ser17 of TRBC1^{∗}01 or TRBC2^{∗}01 exon 1;
Thr45 of TRAC^{∗}01 exon 1 andAsp59 ofTRBC1^{∗}01 or TRBC2^{∗}01 exon 1;
Ser15 of TRAC^{∗}01 exon 1 and Glu15 of TRBCI^{∗}01 or TRBC2^{∗}01 exon 1;
Arg53 of TRAC^{∗}01 exon 1 and Ser54 of TRBC1^{∗}01 or TRBC2^{∗}01 exon 1;
Pro89 of TRAC^{∗}01 exon 1 and Ala19 of TRBC1^{∗}01 orTRBC2^{∗}01 exon 1; and
Tyr10 of TRAC^{∗}01 exon 1 and Glu20 of TRBC1^{∗}01 or TRBC2^{∗}01 exon 1.

In another preferred embodiment, the TCR is a single-stranded TCR.

In another preferred embodiment, the single-stranded TCR is an alpha chain variable domain linked to a beta chain variable domain via a flexible short peptide sequence (linker).

In another preferred embodiment, the single-stranded TCR has an amino acid sequence as set forth in SEQ ID NOs: 6 to 9.

In another preferred embodiment, the alpha chain and/or the beta chain of the TCR is bound with a conjugate at the C- or N-terminus.

In another preferred embodiment, the conjugate is a detectable label, a therapeutic agent, a PKmodifying moiety, or a combination of any of these substances.

In another preferred embodiment, the therapeutic agent that is bound to the TCR is an anti-CD3 antibody attached to the C-or N-terminus of the alpha chain and/or the beta chain of the TCR.

In a second aspect of the present disclosure, there is provided a multivalent TCR complex including at least two TCR molecules, wherein at least one of the TCR molecules is a TCR according to the first aspect of the present disclosure.

In a third aspect of the present disclosure, there is provided a nucleic acid molecule including a nucleic acid sequence encoding a TCR of the first aspect of the present disclosure or a TCR complex of the second aspect of the present disclosure, or a complementary sequence thereto.

In a fourth aspect of the present disclosure, there is provided a vector including a nucleic acid molecule of the third aspect of the present disclosure; preferably, the vector is a viral vector; more preferably, the vector is a lentiviral vector.

In a fifth aspect of the present disclosure, there is provided a host cell including a vector of the fourth aspect of the present disclosure or having a genome into which an exogenous nucleic acid molecule of the third aspect of the present disclosure is integrated.

In a sixth aspect of the present disclosure, there is provided an isolated cell, which is transduced with a nucleic acid molecule of the third aspect of the present disclosure or a vector of the fourth aspect of the present disclosure, or which expresses a TCR of the first aspect of the present disclosure; preferably, the cell is a T cell, NK cell or NKT cell.

In a seventh aspect of the present disclosure, there is provided a pharmaceutical composition including a TCR of the first aspect of the present disclosure, a TCR complex of the second aspect of the present disclosure, or a cell of the sixth aspect of the present disclosure, and a pharmaceutically acceptable carrier.

In an eighth aspect of the present disclosure, there is provided use of a T cell receptor of the first aspect of the present disclosure, a TCR complex of the second aspect of the present disclosure, or a cell of the sixth aspect of the present disclosure, in manufacturing a medicament for treating a tumor or autoimmune disease; preferably the tumor is an HPV positive tumor, such as an HPV16 E6 positive tumor, more preferably the tumor is cervical cancer.

In a ninth aspect of the present disclosure, there is provided a T cell receptor of the first aspect of the present disclosure, a TCR complex of the second aspect of the present disclosure, or a cell of the sixth aspect of the present disclosure, for use as a medicament for treating a tumor or autoimmune disease; preferably the tumor is an HPV positive tumor, such as an HPV16 E6 positive tumor, more preferably the tumor is cervical cancer.

In a tenth aspect of the present disclosure, there is provided a method for treating a disease including administering to a subject in need thereof an appropriate amount of T cell receptor of the first aspect of the present disclosure, a TCR complex of the second aspect of the present disclosure, a cell of the sixth aspect of the present disclosure, or a pharmaceutical composition of the seventh aspect of the present disclosure; preferably, the disease is an HPV positive tumor, and more preferably the tumor is cervical cancer.

In an eleventh aspect of the present disclosure, there is provided a method for preparing a T cell receptor of the first aspect of the present disclosure, including the steps of:
(i) culturing a host cell of the fifth aspect of the present disclosure to express a T cell receptor of the first aspect of the present disclosure; and
(ii) isolating or purifying the T cell receptor.

It is to be understood that within the scope of the present disclosure, the various technical features of the present disclosure and the technical features specifically described hereinafter (as in the Examples) may be combined with each other to constitute a new or preferred technical solution, which will not be repeated herein one by one.

### BRIEF DESCRIPTION OF THE DRAWINGS

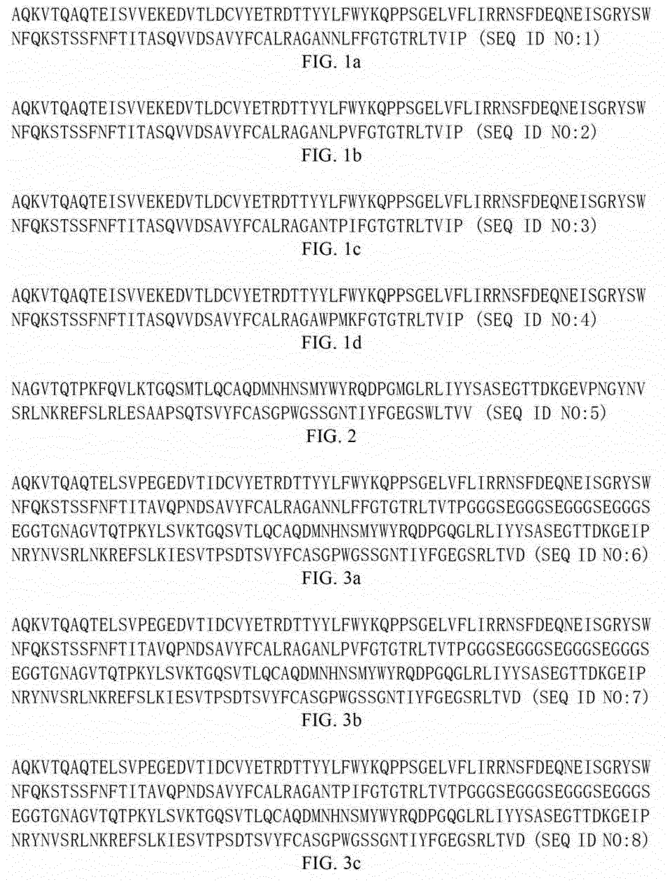

FIGs. 1a-1d show the amino acid sequences of the alpha chain variable domains of the TCR of the present disclosure, respectively, which are capable of specifically binding to the TIHDIILECV-HLA A0201 complex.
FIG. 2 shows the amino acid sequence of the beta chain variable domains of the TCR of the present disclosure, which is capable of specifically binding to the TIHDIILECV-HLAA0201 complex.
FIGs. 3a-3d show the amino acid sequences of the single-stranded TCR of the present disclosure, respectively.
FIGs. 4a-4d show the amino acid sequences of the alpha chain of the soluble TCR of the present disclosure, respectively, wherein the cysteine residues after the mutation are indicated in bold letters.
FIG. 5 shows the amino acid sequence of the beta chain of the soluble TCR of the present disclosure, respectively, wherein the cysteine residues after the mutation are indicated in bold letters.
FIGs. 6a and 6b show the nucleotide sequence encoding the alpha chain variable domains of the TCR of the present disclosure (the amino acid sequence as set forth in SEQ ID NO: 1) and the nucleotide sequence encoding beta chain variable domains of the TCR of the present disclosure (the amino acid sequence as set forth in SEQ ID NO: 5), respectively.
FIGs. 7a-7d show the binding curves of the soluble TCR1, TCR2, TCR3, TCR4 of the present disclosure to TIHDIILECV-HLA A0201 complexes, respectively.
FIG. 8 shows the results of an activation function assay for effector cells transfected with the TCR of the present disclosure against T2 cells loaded with a short peptide.
FIGs. 9a-9b shows the results of an activation function assay for effector cells transfected with the TCR of the present disclosure against tumor cell lines.
FIG. 10 shows the results of a killing function LDH assay for effector cells transfected with the TCR of the present disclosure against T2 cells loaded with a short peptide in gradient.
FIG. 11 shows the results of a killing function LDH assay for effector cells transfected with the TCR of the present disclosure against tumor cell lines.
FIG. 12 shows the results of a killing function IncuCyte assay for effector cells transfected with the TCR of the present disclosure against tumor cell lines.
FIG. 13 is a graph showing the results of double-positive staining for tetramer and anti-CD8-APC.

### DETAILED DESCRIPTION

Through extensive and in-depth research, the present disclosure provides a high-affinity T cell receptor (TCR) that specifically recognizes and binds to the TIHDIILECV-HLA A0201 complex. The TCR has an alpha chain variable domain and a beta chain variable domain. The alpha chain variable domain has three CDRs having amino acid sequences as follows:
CDR1α: TRDTTYY (SEQ ID NO:20),
CDR2α: RNSFDEQN (SEQ ID NO:21), and
CDR3α: ALRAGANNLF (SEQ ID NO: 22), respectively, and
CDR3α includes at least one of the following mutations:

| Residue before mutation | Residue after mutation |
|---|---|
| N at position 7 of CDR3α | W |
| N at position 8 of CDR3α | L or T or P |
| L at position 9 of CDR3α | P or M |
| F at position 10 of CDR3α | V or I or K. |

The beta chain variable domain has three CDRs having amino acid sequences as follows:
β CDR1- MNHNS (SEQ ID NO: 17),
β CDR2- SASEGT (SEQ ID NO:18), and
β CDR3- ASGPWGSSGNTIY (SEQ ID NO: 19), respectively.

Before the present disclosure is described, it is to be understood that the present disclosure is not limited to the specific methods and experimental conditions described, as such methods and conditions may vary. It is also understood that the terminology used herein is for the purpose of describing particular embodiments, and is not intended to be limiting, and the scope of the present disclosure shall be limited only by the appended claim set.

Unless otherwise defined, all technical and scientific terms used herein have the same meaning as commonly understood by those of ordinary skill in the art to which this invention belongs.

While any methods and materials similar or equivalent to those described herein may be used in the practice or testing of the present disclosure, preferred methods and materials are exemplified herein. Terms

### T cell receptor (TCR)

International Immunogenetics Information System (IMGT) can be used to describe a TCR. A native αβ heterodimeric TCR has an alpha chain and a beta chain. Generally speaking, each chain includes a variable (V) region, a junction (J) region and a constant (C) region, and the β chain typically further contains a short diversity (D) region between the variable region and junction region, which however is often considered as a part of the junction region. The junction region of a TCR is determined by the unique TRAJ and TRBJ of IMGT, and the constant region of a TCR is determined by TRAC and TRBC of IMGT.

In IMGT nomenclature, the different numbering of TRAV and TRBV refers to different types of Vα and Vβ, respectively. In the IMGT system, alpha chain constant domain is represented by a symbol TRAC*01, where "TR" represents T cell receptor gene; "A" represents alpha chain gene; C represents constant region; "*01" represents allele gene 1. Beta chain constant domain is represented by a symbol TRBC1^{∗}01 or TRBC2*01, where "TR" represents T cell receptor gene; "B" represents beta chain gene; C represents constant region; "*01" represents allele gene 1. The alpha chain has a uniquely defined constant region, while the beta chain has two possible forms of constant region genes "C1" and "C2". A skilled person in the art can obtain constant region gene sequences of TCR alpha and beta chains from the disclosed IMGT database.

The alpha and beta chains of TCR are generally considered as each having two "domains", i.e., variable domain and constant domain. The variable domain is composed of a variable region linked to a junction region. Therefore, in the specification and claims of the present application, "TCR alpha chain variable domain" refers to a TRAV region linked to TRAJ region, and likewise, "TCR beta chain variable domain" refers to a TRBV region linked to TRBD/TRBJ region.

Each variable region includes three CDRs (complementarity determining regions), CDR1, CDR2, and CDR3, which are embedded in framework sequences. The three CDRs of the alpha chain variable domain of the TCR are CDR1α, CDR2α, and CDR3α, respectively; and the three CDRs of the beta chain variable domain of the TCR are CDR1β, CDR2β and CDR3β, respectively. The framework sequences of TCR variable domains of the present disclosure may be of murine or human origin, preferably of human origin. The constant domain of TCR includes an intracellular portion, transmembrane region, and extracellular portion.

The TCR sequences used in the present disclosure are of human origin. In the present disclosure, the terms "polypeptide of the present disclosure", "TCR of the present disclosure" and "T cell receptor of the present disclosure" are used interchangeably.

### Natural inter-chain disulfide bonds and artificial inter-chain disulfide bonds

There is a group of disulfide bonds between the Cα and Cβ chains in the membrane-proximal region of a native TCR, which is named herein the "natural inter-chain disulfide bond". In the present disclosure, an inter-chain covalent disulfide bond that is artificially introduced and is in a different location from a natural inter-chain disulfide bond is named "artificial inter-chain disulfide bond".

For the convenience of description, in the present disclosure, the positions of the amino acid sequences of TRAC*01 and TRBC1^{∗}01 or TRBC2*01 are sequentially numbered in order from N-terminus to C-terminus. For example, the 60^{th} amino acid in the order from N-terminus to C-terminus in TRBC1^{∗}01 or TRBC2*01 is P (valine), which can be described as Pro60 in TRBC1^{∗}01 or TRBC2^{∗}01 exon 1 in the present disclosure, and can also be expressed as the amino acid at position 60 in TRBC1^{∗}01 or TRBC2^{∗}01 exon 1; for another example, the 61^{st} amino acid in the order from N-terminal to C-terminal in TRBC1^{∗}01 or TRBC2^{∗}01 is Q (glutamine), which can be described as Gln61 of TRBC1^{∗}01 or TRBC2^{∗}01 exon 1 in the present disclosure, and can also be expressed as the amino acid at position 61 of TRBC1^{∗}01 or TRBC2*01 exon 1, and so on. In the present disclosure, the positions of the amino acid sequences of variable regions TRAV and TRBV are numbered according to the positions listed in IMGT. As for an amino acid in TRAV, the position is numbered as 46 in IMGT, which is described in the present disclosure as the amino acid at position 46 of TRAV, and so on. In the present disclosure, if the sequence positions of other amino acids are specifically described, the special description shall prevail.

### Tumors

The term "tumor" refers to include all types of cancer cell growth or carcinogenic processes, metastatic tissues or malignantly transformed cells, tissues or organs, regardless of pathological type or stage of infiltration. Examples of tumors include, without limitation, solid tumors, soft tissue tumors, and metastatic lesions. Examples of solid tumors include malignant tumors of different organ systems, such as sarcoma, lung squamous cell carcinoma, and cancer. For example, infected prostate, lung, breast, lymph, gastrointestinal (e.g., colon) and genitourinary tract (e.g., kidney, epithelial cells), pharynx. T cells transduced with the TCR of the present disclosure can be used to treat any HPV16 E6-related disease presenting the TIHDIILECV-HLA A0201 complex as a HPV16 E6 antigen short peptide, including but not limited to tumors, such as cervical cancer, head and neck tumors, and the like.

### Detailed Description of the present disclosure

During antigen processing, the antigen is degraded within the cell and then carried to the cell surface by the MHC molecule. T cell receptors are capable of recognizing peptide-MHC complexes on the surface of antigen-presenting cells. Thus, in a first aspect of the present disclosure, there is provided a TCR having an activity to bind to the TIHDIILECV-HLA A0201 complex and having an alpha chain variable domain having an amino acid sequence having at least 90% sequence homology to the amino acid sequence as set forth in SEQ ID NO: 1; and/or a beta chain variable domain having an amino acid sequence having at least 90% sequence homology to the amino acid sequence as set forth in SEQ ID NO: 5.

The alpha chain variable domain of the TCR of the present disclosure has an amino acid sequence having at least 90%, preferably 95% (e.g., that may be at least 91%, 92%, 93%, 94%, 95%, 96%, 97%, 98%, 99%) sequence identity to SEQ ID NO: 1; and the beta chain variable domain of the TCR of the present disclosure has an amino acid sequence having at least 90%, preferably 95% (e.g., that may be at least 91%, 92%, 93%, 94%, 95%, 96%, 97%, 98%, 99%) sequence identity to SEQ ID NO: 5. In another preferred embodiment, the TCR in the present disclosure is of human origin.

In a preferred embodiment of the present disclosure, the TCR of the present disclose includes the alpha chain variable domain having an amino acid sequence as set forth in one of SEQ ID NOs:1 to 4; and/or the beta chain variable domain of TCR having an amino acid sequence as set forth in SEQ ID NO:5.

Recognition of pMHC (antigen peptide-major histocompatibility complex) by TCR involves two kinds of binding: binding of TCR to MHC molecules and binding of TCR to polypeptide antigens. The binding surfaces of the TCR are from six regions: CDR1, CDR2, and CDR3 of each of the TCR alpha and beta chains. CDR1 and CDR2 mainly bind to the relatively conserved MHC molecule, while CDR3 mainly binds to the variable antigen polypeptides. This mode of binding provides a good guarantee for the specific binding of TCR to MHC molecules and enables the recognition of variable antigens. (Chinese journal of cell biology 2011, 33(9):955-963). Among them, CDR3 varies most greatly, which directly determines the antigen-binding specificity of TCR. The CDRs, determined by sequencing, of the TCR in the present disclosure are as follows:

| CDR No. | CDR1α | CDR2α | CDR3α | CDR1β | CDR2β | CDR3β |
|---|---|---|---|---|---|---|
| 1 | TRDTTYY | RNSFDEQN | ALRAGANNLF | MNHNS | SASEGT | ASGPWGSSGNTIY |
| 2 | TRDTTYY | RNSFDEQN | ALRAGANLPV | MNHNS | SASEGT | ASGPWGSSGNTIY |
| 3 | TRDTTYY | RNSFDEQN | ALRAGANTPI | MNHNS | SASEGT | ASGPWGSSGNTIY |
| 4 | TRDTTYY | RNSFDEQN | ALRAGAWPMK | MNHNS | SASEGT | ASGPWGSSGNTIY |

The above-mentioned amino acid sequence of the CDR region of the present disclosure can be inserted into any suitable framework structure to prepare a chimeric TCR. In the art, substitutions with amino acids having similar properties generally do not alter the function of the protein. Also, the addition of one or more amino acids at the C-terminus and/or the N-terminus does not generally alter the structure and function of the protein. Thus, the TCR of the present disclosure also encompass a TCR of the present disclosure that has up to five, preferably up to three, more preferably up to two, and most preferably one amino acid (especially an amino acid situated outside the CDRs), substituted with amino acids having similar properties and is still capable of maintaining its functionality.

In a preferred embodiment of the present disclosure, the constant domain of the TCR molecule of the present disclosure is a human constant domain. A person skilled in the art is aware of, or may obtain, the amino acid sequence of the human constant domain by consulting related books or published databases of IMGT(International Immunogenetics Information System). For example, the sequence of the alpha chain constant domain of the TCR molecule of the present disclosure may be "TRAC*01", and the sequence of the beta chain constant domain of the TCR molecule may be "TRBC1*01" or "TRBC2*01". The position 53 of the amino acid sequence given in TRAC*01 of IMGT is Arg, denoted herein as Arg53 of Exon 1 of TRAC*01, and so forth.

In another preferred embodiment, the TCR is an αβ heterodimeric TCR, preferably having an alpha chain constant region sequence TRAC*01 and a beta chain constant region sequence TRBC1*01 or TRBC2*01.

In a preferred embodiment of the present disclosure, the TCR molecule of the present disclosure is a single-stranded TCR molecule composed of part or all of the alpha chain and/or part or all of the beta chain. The description for single-stranded TCR molecules can be found in Chung et al (1994) Proc. Natl. Acad. Sci. USA 91, 12654-12658. According to the literature, one skilled in the art can easily construct a single-stranded TCR molecule including the CDRs region of the present disclosure. In particular, the single-stranded TCR molecules include Vα, Vβ, and Cβ, preferably linked in the order from the N-terminus to the C-terminus.

For the purposes of the present disclosure, the TCR of the present disclosure is a moiety having at least one alpha and/or beta chain variable domain of TCR. They usually include both of the alpha chain variable domain of TCR and the beta chain variable domain of TCR. They may be αβ heterodimers or single-chain forms or any other stable forms. In adoptive immunotherapy, the full-length chain of the αβ heterodimeric TCR (including the cytoplasmic and transmembrane domains) can be used for transfection. The TCR of the present disclosure can be used as a targeting agent for delivering a therapeutic agent to an antigen-presenting cell or used in combination with other molecules to prepare a bifunctional polypeptide to direct effector cells, in which case the TCR is preferably in a soluble form.

Naturally occurring TCR is a membrane protein that is stabilized by its transmembrane region. Like immunoglobulins (antibodies) as antigen recognition molecules, TCR can also be developed for use in diagnosis and treatment, in which case soluble TCR molecules need to be obtained. Soluble TCR molecule does not include their transmembrane regions. Soluble TCR has a wide application, not only for studying the interaction between TCR and pMHC, but also as a diagnostic tool for the detection of infection or as a marker of autoimmune disease. Similarly, soluble TCR may be used to deliver therapeutic agents (e.g., cytotoxin compounds or immunostimulatory compounds) to specific antigen-presenting cells. In addition, soluble TCR may bind to other molecules (e.g., an anti-CD3 antibody) to redirect T cells, which thereby target to specific antigen-presenting cells.

To obtain the soluble TCR, the TCR of the present disclosure may, in one aspect, be a TCR in which an artificial disulfide bond is introduced between the residues of the alpha and beta chain constant domains. Cysteine residues form an artificial interchain disulfide bond between the alpha and beta chain constant domains of the TCR. A cysteine residue can be substituted for other amino acid residues at a suitable position in a native TCR to form an artificial interchain disulfide bond. For example, cysteine residues replacing Thr48 of TRAC*01 exon 1 and Ser57 of TRBC1^{∗}01 or TRBC2^{∗}01 exon 1 can form a disulfide bond. Other sites for introducing cysteine residues to form a disulfide bond may be Thr45 of TRAC*01 exon 1 and Ser77 of TRBC1^{∗}01 or TRBC2*01 exon 1; Tyr10 of TRAC*01 exon 1 and Ser17 of TRBC1^{∗}01 or TRBC2*01 exon 1; Thr45 of TRAC*01 exon 1 and Asp59 of TRBC1^{∗}01 or TRBC2*01 exon 1; Ser15 of TRAC*01 exon 1 and Glu15 of TRBC1^{∗}01 or TRBC2*01 exon 1; Arg53 of TRAC^{∗}01 exon 1 and Ser54 of TRBC1^{∗}01 or TRBC2*01 exon 1; Pro89 of TRAC^{∗}01 exon 1 and Ala19 of TRBC1^{∗}01 or TRBC2*01 exon 1; or Tyr10 of TRAC*01 exon 1 and Glu20 of TRBC1^{∗}01 or TRBC2*01 exon 1. That is, cysteine residues are substituted for any group of the above-mentioned sites in alpha and beta chain constant domains. One or more C-termini of the constant domain of the TCR of the present disclosure may be truncated by up to 50, up to 30, up to 15, up to 10, or up to 8 or fewer amino acids to exclude cysteine residues to achieve the purpose of deleting native disulfide bonds. Alternatively, the cysteine residues forming a natural disulfide bond can also be mutated into another amino acids for achieving the above purpose. The present disclosure also provides a soluble TCR specific to the HPV16 E6 antigen short peptide. As the soluble TCR constructed in Example 2 of the present disclosure, the alpha chain variable domain has an amino acid sequence as set forth in one of SEQ ID NOs: 10-13, and the beta chain variable domain has an amino acid sequence as set forth in SEQ ID NO:14.

As described above, the TCR of the present disclosure may include an artificial disulfide bond introduced between residues of its alpha and beta chain constant domains. It should be noted that the artificial disulfide bond introduced as described above can be contained or not contained between the constant domains, and the TCR of the present disclosure may contain a TRAC constant domain sequence and a TRBC1 or TRBC2 constant domain sequence. The TRAC constant domain sequence and the TRBC1 or TRBC2 constant domain sequence of the TCR can be joined by a natural interchain disulfide bond present in the TCR.

In order to obtain the soluble TCR, in another aspect, the TCR of the present disclosure also includes a TCR having a mutation in its hydrophobic core region, and the mutation in hydrophobic core region is preferably mutations capable of increasing the stability of the soluble TCR of the present disclosure, as described in WO 2014/206304. Such a TCR can have a mutation at following positions in the variable domain hydrophobic core: (alpha and/or beta chain) variable region amino acids at positions 11, 13, 19, 21, 53, 76, 89, 91, 94, and/or alpha chain J gene (TRAJ) short peptide amino acid at reciprocal positions 3, 5, 7 and/or β chain J gene (TRBJ) short peptide amino acid at reciprocal positions 2, 4, 6, wherein the positions in the amino acid sequences are numbered according to the position numbers listed in the International Immunogenetics Information System (IMGT). A skilled person in the art will know the above-described international immunogenetic information system and can obtain the position numbers of the amino acid residues of different TCRs in the IMGT based on the database.

The TCR having a mutation in the hydrophobic core region of the present disclosure may be a stable soluble single-chain TCR composed of a TCR alpha chain variable domain linked to a TCR beta chain variable domain via a flexible peptide chain. It should be noted that the flexible peptide chain of the present disclosure may be any peptide chains suitable for linking the alpha chain variable domain and beta chain variable domain of TCR. In a preferred embodiment of the present disclosure, the amino acid sequence of the single-stranded TCR of the present disclosure is selected from the group consisting of SEQ ID NOs: 6 to 9.

Further, in terms of stability, patent NO. 201680003540.2 also discloses that the introduction of artificial interchain disulfide bonds between the alpha chain variable region and the beta chain constant region of TCR can significantly improve the stability of TCR. Thus, an artificial interchain disulfide bond may also be contained between the alpha chain variable region and the beta chain constant region of the TCR of the present disclosure. In particular, the cysteine residues forming an artificial interchain disulfide bond between the alpha chain variable region and the beta chain constant region of the TCR are substituted for amino acid at position 46 of TRAV and amino acid at position 60 of TRBC1^{∗}01 or TRBC2^{∗}01 exon 1; amino acid at position 47 of TRAV and amino acid at position 61 of TRBC1^{∗}01 or TRBC2^{∗}01 Exon 1; amino acid at position 46 of TRAV and amino acid at position 61 of TRBC1^{∗}01 or TRBC2^{∗}01 Exon 1; or amino acid at position 47 of TRAV and amino acid at position 60 of TRBC1^{∗}01 or TRBC2^{∗}01 Exon 1. Preferably, such TCR may include (i) all or part of an alpha chain of the TCR other than its transmembrane domain, and (ii) all or part of a beta chain of the TCR other than its transmembrane domain, wherein (i) and (ii) each include a variable domain chain and at least part of a constant domain of the TCR, and the alpha chain and beta chain form a heterodimer. More preferably, such TCR may include an alpha chain variable domain and a beta chain variable domain as well as all or part of a beta chain constant domain other than the transmembrane domain but no alpha chain constant domain, and the alpha chain variable domain and the beta chain of the TCR form a heterodimer.

Mutation may be carried out by any suitable method including, but not limited to, those based on polymerase chain reaction (PCR), restriction enzyme-based cloning or linkage-independent cloning (LIC) methods. These methods are detailed in many standard molecular biology textbooks. More details about polymerase chain reaction (PCR) mutagenesis and restriction enzyme-based cloning can be found in Sambrook and Russell, (2001) Molecular Cloning-A Laboratory Manual (Third Edition) CSHL Press. More information about LIC methods can be found in Rashtchian, (1995) Curr Opin Biotechnol 6(1): 30-6.

The method for producing the mutation may be, but is not limited to, screening for a TCR that specifically binds to the TIHDIILECV-HLA A0201 complex from a diverse library of phage particles displaying such TCRs, as described in a literature (Li, et al). (2005) Nature Biotech 23(3): 349-354).

The TCR of the present disclosure can be provided in the form of a multivalent complex. The multivalent TCR complex of the present disclosure includes a polymer formed by combining two, three, four or more TCRs of the present disclosure, for example, a tetrameric domain of p53 can be used to produce a tetramer. Alternatively, a plurality of TCRs of the present disclosure can be combined with another molecule to form a complex. The TCR complexes of the present disclosure can be used to track or target specific antigen-presenting cells in vitro or in vivo, and to produce intermediates of other multivalent TCR complexes having such applications.

The TCR of the present disclosure may be used alone or in combination with a conjugate in a covalent manner or other manner, preferably in a covalent manner. The conjugate includes a detectable label (for diagnostic purposes, wherein the TCR is used to verify the presence of a cell presenting TIHDIILECV-HLA A0201 complex), a therapeutic agent, a PK (protein kinase)-modifying moiety, or a combination of any of the above-described substances.

Detectable labels for diagnostic purposes include, but are not limited to, fluorescent or luminescent labels, radioactive labels, MRI (magnetic resonance imaging) or CT (electron computed tomography) contrast agents, or enzymes capable of producing detectable products.

Therapeutic agents that can be combined with or coupled to the TCRs of the present disclosure include, but are not limited to: 1. radionuclides (Koppe et al., 2005, Cancer metastasis reviews 24, 539); 2. biotoxin (Chaudhary et al., 1989, Nature 339, 394; Epel et al., 2002, Cancer Immunology and Immunotherapy 51, 565); 3. cytokines, such as IL-2 and the like (Gillies et al., 1992, National Academy of Sciences (PNAS) 89, 1428; Card et al., 2004, Cancer Immunology and Immunotherapy 53, 345; Halin et al., 2003, Cancer Research 63, 3202); 4. antibody Fc fragment (Mosquera et al., 2005, The Journal Of Immunology 174, 4381); 5. antibody scFv fragments (Zhu et al., 1995, International Journal of Cancer 62, 319); 6. gold nanoparticles/Nanorods (Lapotko et al., 2005, Cancer letters 239, 36; Huang et al., 2006, Journal of the American Chemical Society 128, 2115); 7. viral particles (Peng et al., 2004, Gene therapy 11, 1234); 8. liposomes (Mamot et al., 2005, Cancer research 65, 11631); 9. nanomagnetic particles; 10. prodrug activating enzymes (e.g., DT-diaphorase (DTD) or biphenyl hydrolase-like protein (BPHL); 11. chemotherapeutic agent (e.g., cisplatin) or any form of nanoparticles, and the like.

An antibody or a fragment thereof to which the TCR of the present disclosure binds includes an anti-T cell or an NK-cell determining antibody, such as an anti-CD3 or anti-CD28 or anti-CD16 antibody, and the above antibody or a fragment thereof binds to a TCR, thereby better directing effector cells to target cells. In a preferred embodiment, the TCR of the present disclosure binds to an anti-CD3 antibody or a functional fragment or variant thereof. Specifically, a fusion molecule of the TCR of the present disclosure with an anti-CD3 single-chain antibody includes (1) an alpha chain variable domain of TCR having an amino acid sequence as set forth in one of SEQ ID NOs: 1 to 4, and an beta chain variable domain of TCR having an amino acid sequence as set forth in SEQ ID NO:5; or (2) a single-stranded TCR having an amino acid sequence as set forth in any one of SEQ ID NOs: 6 to 9.

The present disclosure also relates to a nucleic acid molecule encoding the TCR of the present disclosure. The nucleic acid molecule of the present disclosure may be in the form of DNA or RNA. DNA can be a coding strand or a non-coding strand. For example, a nucleic acid sequence encoding the TCR of the present disclosure may be the same as the nucleic acid sequence as set forth in the Figures of the present disclosure or a degenerate variant thereof. By way of example, "degenerate variant", as used herein, refers to a nucleic acid sequence that encodes a protein having a sequence of SEQ ID NO: 1 but is different from the sequence of SEQ ID NO: 15.

The full-length sequence of the nucleic acid molecule of the present disclosure or a fragment thereof can generally be obtained by, but not limited to, PCR amplification, recombinant methods, or synthetic methods. At present, it is possible to obtain a DNA sequence encoding the TCR (or a fragment thereof, or a derivative thereof) of the present disclosure completely by chemical synthesis. The DNA sequence can then be introduced into various existing DNA molecules (or vectors) and cells known in the art.

The present disclosure also relates to a vector including the nucleic acid molecule of the present disclosure, as well as a host cell genetically engineered using the vector or coding sequence of the present disclosure.

The vectors including the nucleic acid molecules of the present disclosure include expression vectors, i.e., constructs capable of expression in vivo or in vitro. Common vectors include bacterial plasmids, bacteriophages and animal and plant viruses. Viral delivery systems include, but are not limited to, adenovirus vectors, adeno-associated virus (AAV) vectors, herpes virus vectors, retroviral vectors, lentiviral vectors, and baculovirus vectors. Preferably, the vector can transfer the nucleotide of the present disclosure into a cell, such as a T cell, such that the cell expresses HPV16 E6 antigen-specific PCR. Ideally, the vector should be capable of sustained high levels of expression in T cells.

The host cell contains a vector of the present disclosure or has a chromosome into which a nucleic acid molecule of the present disclosure is integrated. The host cell is selected from prokaryotic cells and eukaryotic cells, such as Escherichia coli, yeast cells, CHO cells, etc.

In addition, the present disclosure also includes isolated cells expressing the TCR of the present disclosure, which may be T cells, NK cells, NKT cells, etc., preferably T cells. The T cells may be derived from T cells isolated from the subject or may be a mixed population of cells isolated from the subject, such as part of a peripheral blood lymphocyte (PBL) population. For example, the cells may be isolated from peripheral blood mononuclear cells (PBMCs), and may be CD4+ helper T cells or CD8+ cytotoxic T cells. The cells may be in a mixed population of CD4+ helper T cells/CD8+ cytotoxic T cells. Generally, the cells can be activated with antibodies (e.g., anti-CD3 or anti-CD28 antibodies) to make them more susceptible to transfection, e.g., with a vector including a nucleotide sequence encoding a TCR molecule of the present disclosure.

Alternatively, the cells of the present disclosure can also be or be derived from stem cells, such as hematopoietic stem cells (HSC). Transferring a gene to HSC would not result in the expression of TCR on the cell surface, since CD3 molecules are not expressed on the surface of stem cells. However, when stem cells differentiate into lymphoid precursors that migrate to the thymus, the expression of CD3 molecules will initiate the expression of the introduced TCR molecules on the surface of thymocytes.

There are a number of methods suitable for T cell transfection with DNA or RNA encoding the TCR of the present disclosure (e.g., Robbins et al., (2008) J. Immunol. 180: 6116-6131). T cells expressing the TCR of the present disclosure can be used in adoptive immunotherapy. A skilled person in the art can know many suitable methods for performing adoptive therapy (e.g., Rosenberg et al., (2008) Nat Rev Cancer 8(4): 299-308).

The present disclosure also provides a pharmaceutical composition, including a TCR of the present disclosure, a TCR complex of the present disclosure, or a cell presenting the TCR of the present disclosure, and a pharmaceutically acceptable carrier.

The present disclosure also provides a method for treating a disease, including administering to a subject in need thereof an appropriate amount of a TCR of the present disclosure, a TCR complex of the present disclosure, a cell presenting a TCR of the present disclosure, or a pharmaceutical composition of the present disclosure.

The TCR, TCR complex of the present disclosure, or T cells transfected with the TCR of the present disclosure can be provided together with a pharmaceutically acceptable carrier in a pharmaceutical composition. The TCR, multivalent TCR complex, or cell of the present disclosure is typically provided as part of a sterile pharmaceutical composition, which typically includes a pharmaceutically acceptable carrier. The pharmaceutical composition can be in any suitable form (depending on the desired method for administration to a patient). It can be provided in a unit dosage form, usually in a sealed container, which can be provided as part of a kit. Such a kit (but not necessarily) includes instructions. The kit may include a plurality of said unit dosage form.

Furthermore, the TCR of the present disclosure may be used alone or in combination with other therapeutic agents (e.g., formulated in the same pharmaceutical composition).

The pharmaceutical composition may also contain a pharmaceutically acceptable carrier. The term "pharmaceutically acceptable carrier" refers to a carrier for the administration of a therapeutic agent. The term refers to any pharmaceutical carrier that does not itself induce the production of antibodies harmful to the individual receiving the composition, and which may be administered without undue toxicity. Such carriers are well known to those of ordinary skill in the art. A thorough discussion of pharmaceutically acceptable excipients is available in Remington's Pharmaceutical Sciences (Mack Pub. Co., N.J. 1991). Such carriers include, but are not limited to, saline, buffers, glucose, water, glycerol, ethanol, adjuvants, and combinations thereof.

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such carriers.

Typically, the therapeutic compositions may be prepared as injectables, for example, as liquid solutions or suspensions, or may be prepared as solid forms suitable for being formulated into a solution, or suspension, liquid vehicles prior to injection may also be prepared.

Once formulated, the compositions of the present disclosure may be administered by conventional routes, including, but not limited to, intraocular, intramuscular, intravenous, subcutaneous, intradermal, or topical administration, preferably parenteral administration, including subcutaneous, intramuscular, or intravenous administration. The object to be prevented or treated may be an animal; especially human.

When the pharmaceutical composition of the present disclosure is used for actual treatment, pharmaceutical compositions in various dosage forms may be employed depending on the uses, preferably, for example, an injection, an oral preparation, or the like.

These pharmaceutical compositions can be formulated in accordance with conventional methods by mixing, diluting or dissolving, occasionally, with appropriate pharmaceutical additives such as excipients, disintegrators, binders, lubricants, diluents, buffers, isotonicities, antiseptics, moistening agents, emulsifiers, dispersing agents, stabilizing agents and dissolving aids, and the formulation can be performed conventionally depending upon the dosage form.

The pharmaceutical compositions of the present disclosure can be administered in a sustained-release form. For example, the TCR of the present disclosure can be incorporated into a pellet or microcapsule with a sustained-release polymer as a carrier, and the pellet or microcapsule is surgically planted into the tissues to be treated. As examples of sustained-release polymer, ethylene-vinylacatate copolymer, polyhydromethacrylate, polyacrylamide, polyvinylpirrolidone, methylcellulose, lactic acid polymer, lactic acid-glycolic acid copolymer and the like can be illustrated. Preferably, a biodegradable polymer such as lactic acid polymer and lactic acid-glycolic acid copolymer can be illustrated.

The present disclosure provides a method for treating HPV16 E6-related diseases including transfusing into a patient isolated T cells, preferably derived from the patient itself, expressing the TCR of the present disclosure. Generally, the method includes (1) isolating T cells from a patient, (2) transducing T cells in vitro with a nucleic acid molecule of the present disclosure or a nucleic acid molecule capable of encoding a TCR of the present disclosure, and (3) transfusing the genetically engineered T cells into the patient. When the pharmaceutical compositions of the present disclosure are employed as a practical treatment, the amount of the TCR or TCR complex of the present disclosure or the cell presenting the TCR of the present disclosure as the active ingredient may be reasonably determined based on the body weight, age, sex, and degree of symptoms of each patient to be treated, and ultimately by a physician.

In addition, the TCR of the present disclosure may also be a hybrid TCR including sequences derived from more than one species. For example, studies have shown that murine TCR is more effectively expressed in human T cells than human TCR. Thus, the TCR of the present disclosure may include a human variable domain and a murine constant domain. The drawback of this method is that it may trigger an immune response. Therefore, there should be an immunosuppressive regimen to allow the implantation of murineexpressing T cells when it is used in adoptive T cell therapy.

It is to be understood that amino acid designations herein are denoted by the internationally used single letters or three letters and that the correspondence of the single letters of amino acid designations to the three letters is as follows: Ala (A), Arg (R), Asn (N), Asp (D), Cys (C), Gln (Q), Glu (E), Gly (G), His (H), Ile (I), Leu (L), Lys (K), Met (M), Phe (F), Pro (P), Ser (S), Thr (T), Trp (W), Tyr (Y), and Val (V).

In the present disclosure, Pro 60 or 60P each represents proline at position 60. In addition, the specific form of the mutation described in the present disclosure is expressed as, for example, "N93D" representing that N at position 93 is substituted with D, and similarly, "N93D/E" representing that N at position 93 is substituted with D or with E, and so on.

In the art, substitutions with amino acids having similar properties generally do not alter the function of the protein. Also, the addition of one or more amino acids at the C-terminus and/or the N-terminus does not generally alter the structure and function of the protein. Thus, the TCRs of the present disclosure also encompass a TCR of the present disclosure that has up to five, preferably up to three, more preferably up to two, and most preferably one amino acid (especially an amino acid situated outside the CDRs), substituted with amino acids having similar properties and is still capable of maintaining its functionality.

The present disclosure also includes a TCR obtained from the TCR of the present disclosure with slight modifications. Form of modifications (usually without altering the primary structure) include chemically derived forms of the TCR of the present disclosure, such as acetylation or carboxylation. Modifications also include glycosylation, such as those TCRs produced by glycosylation modifications in the synthesis and processing or in further processing steps of the TCR of the present disclosure. Such modification can be accomplished by exposing the TCR to an enzyme exerting glycosylation (such as glycosylase or a deglycosylase from mammals). Modification forms also include sequences having phosphorylated amino acid residues (such as phosphotyrosine, phosphoserine, phosphothreonine). Also included are TCRs that have been modified for enhancing their antiproteolytic properties or optimizing solubility properties.

The present disclosure has the main advantages that:
(1) The TCR of the present disclosure is capable of specifically binding to the HPV16 E6 antigen short peptide complex TIHDIILECV-HLA A0201, and
(2) against the positive target cells, the effector cells transduced with the TCR of the present disclosure can be specifically activated and also have a strong killing function.

The present disclosure is further illustrated by the following specific examples. It is to be understood that these examples are for illustrative purposes only and are not intended to limit the scope of the present disclosure. Further, in the following, the technical scheme in embodiments of the present disclosure will be clearly and completely described in connection with the accompanying drawings of the embodiments of the present disclosure. The described examples are only some, but not all, embodiments of the present disclosure.

The experimental methods in the following examples which do not specify the specific conditions are usually performed under conventional conditions, for example, conditions described in Sambrook and Russell et al., Molecular Cloning-A Laboratory Manual (Third Edition) (2001) CSHL Press, or in accordance with the conditions recommended by the manufacturer. Percentages and parts are calculated by weight unless otherwise stated. The experimental materials used in the examples of the present disclosure can be commercially available unless otherwise specified. E. coli DH5a was purchased from Tiangen, E. coli BL21 (DE3) was purchased from Tiangen, E. coli Tuner (DE3) was purchased from Novagen, and plasmid pET28a was purchased from Novagen.

### Example 1. Acquisition of HPV16 E6 antigen-specific TCR

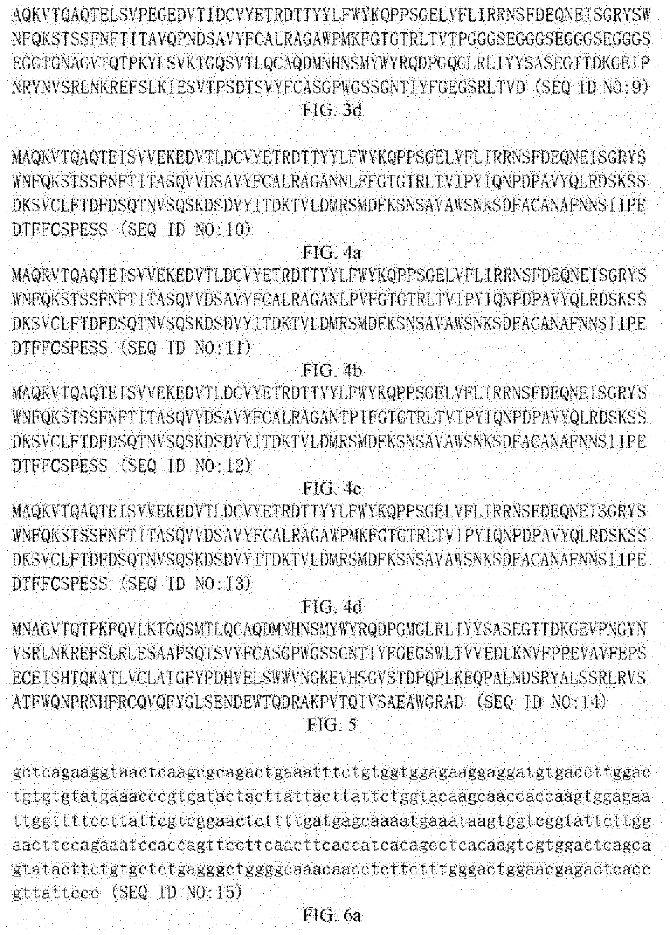

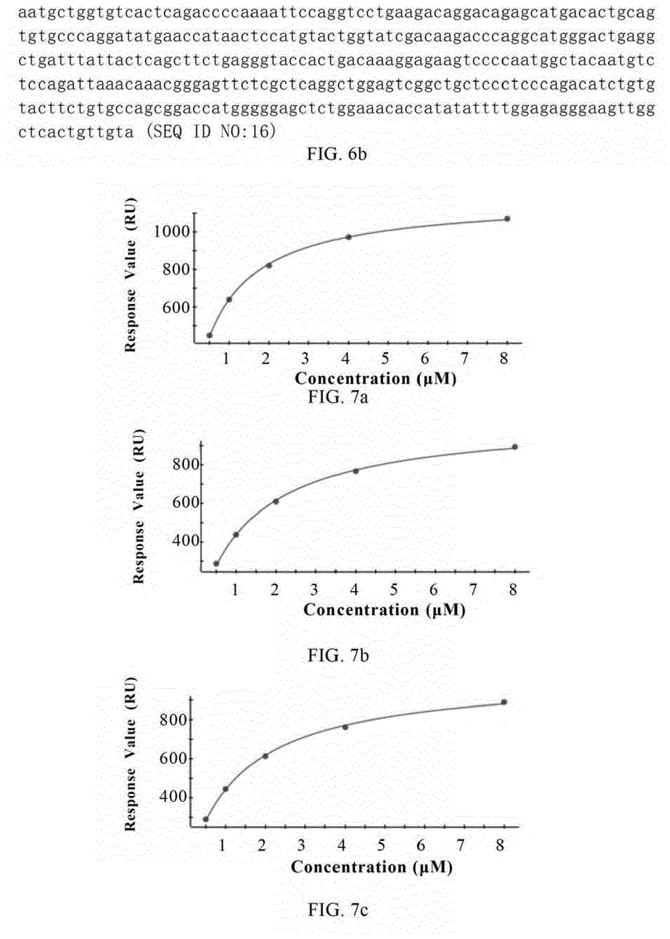

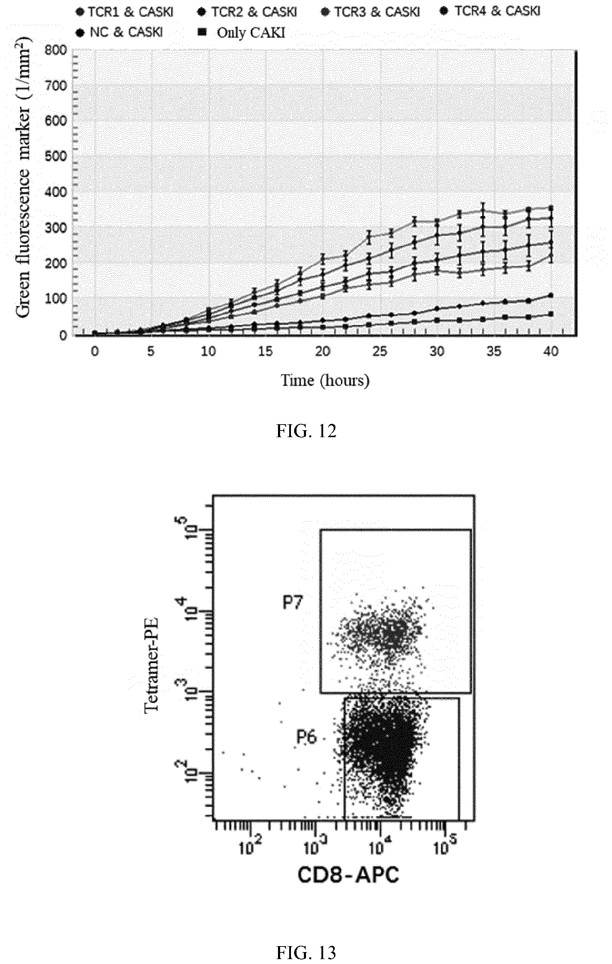

Peripheral blood lymphocytes (PBL) from healthy volunteers with genotype HLA-A0201 were stimulated with a synthetic short peptide TIHDIILECV (GenScript Biotech Corp.). The TIHDIILECV short peptide was renatured with biotin-labeled HLA-A0201 to prepare pMHC haploids. These haploids are combined with PE-labeled streptavidin (BD Company) to form a PE-labeled tetramer. Cells double-positive for both the tetramer and anti-CD8-APC were sorted out. The results of double-positive staining were shown in FIG. 13. After several successful rounds of amplification screening, a T cell receptor capable of specifically binding to the antigen short peptide complex TIHDIILECV-HLA A0201 derived from HPV16 E6 was unexpectedly obtained in the present disclosure. The binding characterization profile is shown in FIG. 7a. Meanwhile, against the HPV E6 positive target cell line, the effector cells transduced with the TCR of the present disclosure can be activated and have a strong killing function. After sequencing, the nucleotide sequences of the alpha chain variable domain (SEQ ID NO: 15) and beta chain variable domain (SEQ ID NO: 16) of TCR which was specific to TIHDIILECV and HLA-A0201 restricted, were shown in FIGs. 6a and 6b, respectively. The amino acid sequence of the alpha chain variable domain of the TCR was as set forth in SEQ ID NO: 1, and the amino acid sequence of the beta chain variable domain of the TCR was as set forth in SEQ ID NO: 5. For the convenience of description, it was named TCR1.

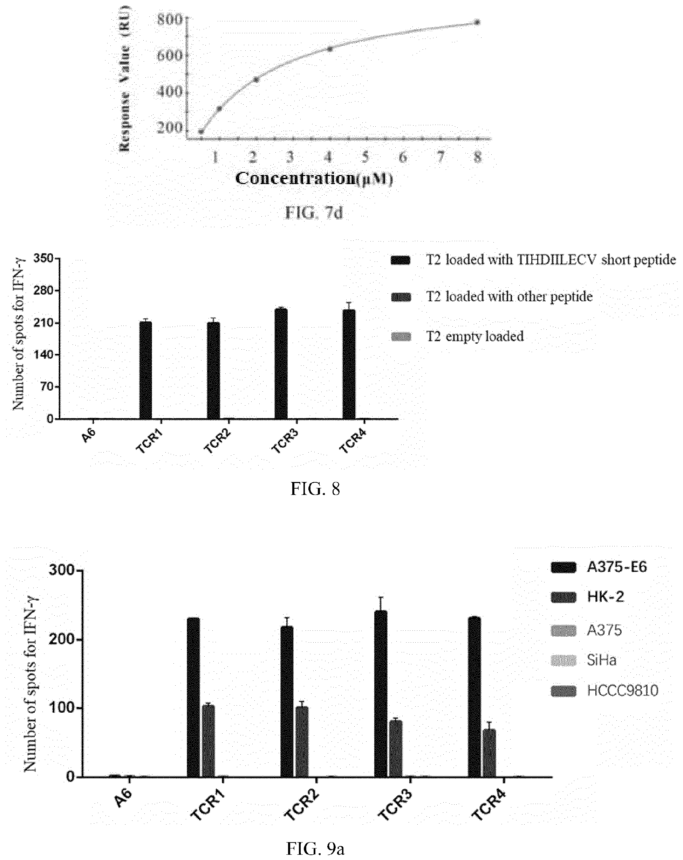

In the present disclosure, we further employed methods of site-directed mutagenesis well known to those skilled in the art, as described in patent document WO2014/206304, to construct a stable single-stranded TCR molecule composed of said alpha chain variable domain linked to said beta chain variable domain via a flexible short peptide (linker), followed by expression, renaturation and purification. A method for displaying and screening TCR phage described by Li et al. (2005) Nature Biotech 23 (3):349-354 was applied to a single-stranded TCR template. After several rounds of panning, a single-stranded TCR, which also binds specifically to the antigen short peptide complex TIHDIILECV-HLA A0201 derived from HPV16 E6, was obtained. The mutations in its CDR region were introduced into the corresponding sites of the variable domain of the αβ heterodimeric TCR so as to obtain TCR2, TCR3 and TCR4. Binding characterization using BIAcore showed that TCR2, TCR3, and TCR4 can also bind specifically to complex TIHDIILECV-HLA A0201, with the binding characterization profile as shown in FIGs. 7b, 7c, and 7d, respectively. In addition, we also surprisingly found that effector cells transduced with TCR2, TCR3, or TCR4 can also be specifically activated and have a strong killing function against the HPV E6 positive target cell line. The alpha chain of TCR2, TCR3 or TCR4 had at least 95% sequence homology to the alpha chain of TCR1, and similarly, the beta chain of TCR2, TCR3 or TCR4 had at least 95% sequence homology to the beta chain of TCR1.

Specifically, TCR2, TCR3 or TCR4 had 3 or 4 mutations in the CDR3 of the alpha chain as compared to TCR1, and the specific CDR is shown in Table 1 below:

**Table 1**

| CDR No. | CDR1α | CDR2α | CDR3α | CDR1β | CDR2β | CDR3β |
|---|---|---|---|---|---|---|
| 1 | TRDTTYY | RNSFDEQN | ALRAGANNLF | MNHNS | SASEGT | ASGPWGSSGNTIY |
| 2 | TRDTTYY | RNSFDEQN | ALRAGANLPV | MNHNS | SASEGT | ASGPWGSSGNTIY |
| 3 | TRDTTYY | RNSFDEQN | ALRAGANTPI | MNHNS | SASEGT | ASGPWGSSGNTIY |
| 4 | TRDTTYY | RNSFDEQN | ALRAGAWPMK | MNHNS | SASEGT | ASGPWGSSGNTIY |

More particularly, the sequences of the alpha and beta chain variable domains of the TCR of the present disclosure were as follows:

**Table 2**

| TCR No. | Sequence of alpha chain variable domain SEQ ID NO: | Sequence of beta chain variable domain SEQ ID NO: |
|---|---|---|
| 1 | 1 | 5 |
| 2 | 2 | 5 |
| 3 | 3 | 5 |
| 4 | 4 | 5 |

The amino acid sequence of the alpha chain variable domain of the TCR was as set forth in any one of SEQ ID NOs: 1 to 4; and/or the amino acid sequence of the beta chain variable domain of the TCR was as set forth in SEQ ID NO: 5.

### Example 2. Expression, refolding and purification of soluble TCR

In this example, a cysteine residue was introduced into the constant domains of the alpha and beta chains of the TCR of the present disclosure, respectively, to form an artificial interchain disulfide bond so as to obtain a stable soluble TCR molecule, in order to assess the interaction between the TCR and the TIHDIILECV-HLA A0201 complex. The amino acid sequence of the alpha chain was as set forth in one of SEQ ID NOs: 10-13, as shown in FIGs.4a-4d; and the amino acid sequence of the beta chain thereof was as set forth in SEQ ID NO: 14, as shown in FIG.5.

The target gene sequences of the aforementioned TCR alpha and beta chains were synthesized and inserted into expression vectors pET28a+ (Novagene) respectively by the standard method described in Sambrook and Russell, Molecular Cloning-A Laboratory Manual (Third Edition). The upstream and downstream cloning sites were NcoI and NotI, respectively. The inserted fragment was confirmed correct by sequencing.

The expression vectors for the alpha and beta chains of TCR were transformed into the expressing bacteria BL21 (DE3) by chemical transformation method. The bacteria were grown in LB culture medium and induced at OD600 value of 0.6 with IPTG at a final concentration of 0.5 mM. The inclusion bodies formed after expression of the alpha and beta chains of TCR were extracted with BugBuster Mix (Novagene) and washed repeatedly with BugBuster solution. The inclusion bodies were finally dissolved in 6 M guanidine hydrochloride, 10 mM dithiothreitol (DTT), 10 mM ethylenediamine tetraacetic acid (EDTA), and 20 mM Tris (pH 8.1).

The dissolved alpha and beta chains of TCR were quickly mixed at a mass ratio of 1:1 in 5 M urea, 0.4M arginine, 20 mM Tris (pH 8.1), 3.7 mM cystamine, 6.6 mM β-mercapoethylamine (4°C), with the final concentration of 60 mg/mL. After mixing, the solution was dialyzed in 10 volumes of deionized water (4°C). After 12 hours the deionized water was replaced with a buffer solution (20 mM Tris, pH 8.0) and continued dialysis at 4°C for 12 hours. The solution after dialysis was filtered through a 0.45 µM filter membrane and purified using an anion exchange column (HiTrap Q HP, 5 ml, GE Healthcare). Whether the elution peaks contain TCR having alpha-beta dimer that has been successfully renatured was confirmed by SDS-PAGE gel. TCR was subsequently further purified by gel filtration chromatography (HiPrep 16/60, Sephacryl S-100 HR, GE Healthcare). The purified TCR has purity of greater than 90% as determined by SDS-PAGE and has a concentration as determined by BCA method.

### Example 3. Characterization of binding

In this example, the binding activity of the soluble TCR molecule obtained according to Example 2 to the TIHDIILECV-HLA A0201 complex was tested using a BIAcore T200 real-time analysis system. The TIHDIILECV-HLA A0201 complex was prepared by methods well known to those skilled in the art, mainly including purification, renaturation, repurification, and biotinylation. The anti-streptavidin antibody (GenScript) was added to the coupling buffer (10 mM sodium acetate buffer, pH 4.77), and then the antibody was passed through a CM5 chip previously activated with EDC and NHS so that the antibody was immobilized on the chip surface, and finally the unreacted activated surface was blocked with a solution of ethanolamine in hydrochloric acid to complete the coupling. The coupling was at a level of about 15,000 RU. A low concentration of streptavidin was allowed to flow through the surface of the chip coated with the antibody, then the TIHDIILECV-HLA A0201 complex was allowed to flow through a detection channel, with the other channel serving as a reference channel. Then 0.05 mM of biotin was allowed to flow through the chip at a flow rate of 10 µL/min for 2 minutes, blocking the remaining binding site for streptavidin.

The kinetic parameters were calculated using the BIAcore Evaluation software to obtain the binding kinetic profiles of the soluble TCR molecules of the present disclosure binding to the TIHDIILECV-HLA A0201 complex, respectively, as shown in FIGs.7a-7d. The profiles showed that all the soluble TCR molecules of the present disclosure can bind to the TIHDIILECV-HLA A0201 complex. Also, the binding activity of the soluble TCR molecule of the present disclosure to several complexes of other unrelated antigen short peptides and HLA was also tested by the method. The results showed that the TCR molecule of the present disclosure would not bind to other unrelated antigens.

### Example 4. Activation function assay of effector cells transfected with TCRs of the present disclosure against short peptide-loaded target cells

IFN-gamma is a potent immunomodulator produced by activated T lymphocytes. Thus in this example, the number of IFN-gamma was counted by ELISPOT assay which is well known to those skilled in the art to verify the activation function and antigen specificity of the cells transfected with TCR of the present disclosure. The CD3+ T cells isolated from the blood of healthy volunteers were transfected with the TCR of the present disclosure and were then used as effector cells, and CD3+ T cells from the same volunteer transfected with other TCRs (A6, TCRs against other antigens) were used as control. The numbering of the TCR and the sequences of the alpha and beta variable domains thereof were shown in Table 2. Target cells used are T2 cells loaded with the HPV16 E6 antigen short peptide TIHDIILECV, loaded with other peptides, or unloaded.

The procedure of the assay was as follows. At first, an ELISPOT plate was prepared. The ELISPOT plate was ethanol-activated and coated at 4°C overnight. On day 1 of the assay, the coating solution was removed, and the plate was washed and blocked by incubating for two hours at room temperature. After the blocking solution was removed, each of the components in the assay was added to the ELISPOT plate, wherein target cells were 1×10⁴ cells/well, effector cells were 2×10³ cells/well (calculated based on the positive rate of transfection), a final concentration of the short peptide was 1×10⁻⁶ M/well. The assay was performed in duplicate. Incubation was performed overnight (37°C, 5% CO2). On day 2 of the assay, the plate was washed, subjected to secondary detection and color development, and then dried. Spots formed on the films were counted using the immunoblotted plate reader (ELISPOT READER System; AID 20 Company).

The experimental results were shown in Figure 8. Against the target cells loaded with the HPV16 E6 antigen short peptide TIHDIILECV, the effector cells transfected with the TCR of the present disclosure had an obvious activation effect, while the effector cells transfected with other TCRs had no activity; at the same time, the effector cells transfected with the TCR of the present disclosure had no response to target cells loaded with other peptides or unloaded.

### Example 5. Activation function assay of effector cells transfected with TCR of the present disclosure against tumor cell lines

To revalidate the activation function and specificity of effector cells transfected with the TCR of the present disclosure, two rounds of ELISPOT assays were performed using a tumor cell line in this example. The CD3+ T cells isolated from the blood of healthy volunteers were transfected with the TCR of the present disclosure and were then used as effector cells, and CD3+ T cells from the same volunteer transfected with other TCRs (A6) were used as the negative control. The numbering of the TCR and the sequences of the alpha and beta variable domains thereof were shown in Table 2. In the first round of assay, A375-E6 (HPV16 E6 overexpression), and HK-2 were used as the HPV16 E6 positive tumor cell lines, and A375, SiHa, and HCCC9810 as the negative tumor cell lines. In the second round of assay, the A375-E6 (HPV16 E6 overexpression) were used as HPV16 E6 positive tumor cell lines, and A375, KATOIII, MDA-MB-231, MEL526, and LCL as the negative tumor cell lines.

The procedure of the assay was described in Example 4, wherein each of the components was added to the ELISPOT plate, wherein the target cells were 2×10⁴ cells/well and the effector cells were 2×10³ cells/well (calculated based on the positive rate of transfection).

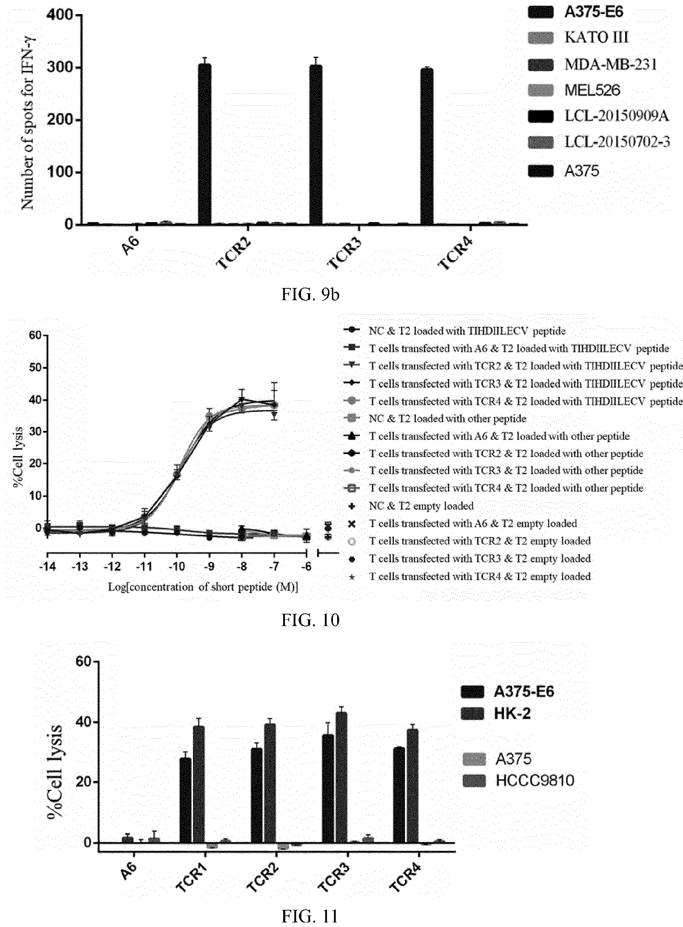

As shown in FIGs.9a and 9b, the effector cells transfected with the TCR of the present disclosure have an obvious activation effect against the HPV16 E6 positive tumor cell lines, while the effector cells transfected with the other TCRs have no activity. Also, the effector cells transfected with the TCR of the present disclosure are substantially inactive against the HPV16 E6 negative tumor cell lines.

### Example 6. Killing function LDH assay of effector cells transfected with TCR of the present disclosure against T2 cells loaded with a short peptide in gradient

Lactodehydrogenase (LDH), which is abundant in the cytosol, normally cannot pass through the cell membrane but can be released out of the cell when the cell is injured or dead. At this time, the LDH activity in the cell culture solution is directly proportional to the number of dead cells. In this example, the killing function of the cells transfected with the TCR of the present disclosure was verified by assaying the release of the LDH by non-radioactive cytotoxicity assays well known to those skilled in the art. This assay is a colorimetric substitution assay for the 51Cr release cytotoxicity assay, which quantitatively measures the amount of LDH released after cell lysis. The LDH released in the culture medium was measured utilizing a 30-minute coupled enzyme reaction, in reaction the LDH converts a tetrazolium salt (INT) to red formazan. The amount of red product produced is proportional to the number of lysed cells. The visible light absorbance data at 490 nm can be collected using a standard 96-well plate reader. Calculation formula: %Cytotoxicity=100% × (experiment-effector cell spontaneous-target cell spontaneous) / (target cell maximum-target cell spontaneous).

In the LDH experiment in this example, the CD3+ T cells isolated from the blood of healthy volunteers were transfected with the TCR of the present disclosure and were then used as effector cells, and CD3+ T cells of the same volunteer transfected with other TCRs (A6) or empty transfected (NC) were used as controls. The numbering of the TCR and the sequences of the alpha and beta variable domains thereof were shown in Table 2. Target cells used were T2 cells loaded with TIHDIILECV peptide, while T2 cells loaded with other antigens or unloaded were used as controls.

The procedure of the assay was as follows. LDH plate was prepared at first, 3^{∗}10⁴ cells/well for the target cells and 3^{∗}10⁴ cells/well for the effector cells (calculated based on the positive rate of transfection) were added to the corresponding wells. Then, in the experimental group, HPV16 E6 antigen short peptide TIHDIILECV was added so that the final concentration of the short peptide in the plate was 1×10⁻¹⁴M to 1×10⁻⁶M, with a total of 9 gradients. In the control group, other short peptides were added so that the final concentration of the short peptides was 1×10⁻⁸M to 1×10⁻⁶M, with a total of 3 gradients. The assay was performed in triplicate. Meanwhile, an effector cell spontaneous well, a target cell spontaneous well, a target cell maximum well, a volume calibration control well and a culture medium background control well were set. Incubation was performed overnight (37°C, 5% CO2). On day 2 of the assay, color development was measured. The absorption value was recorded at 490 nm with a plate reader (Bioteck) once the reaction was stopped.

As shown in FIG.10, the effector cells transfected with the TCR of the present disclosure have a strong killing function against target cells loaded with HPV16 E6 antigen short peptide TIHDIILECV, and can be effective even when the above short peptide is at a low concentration, while the effector cells transfected with other TCRs or empty transfected have no killing effect from beginning to end. At the same time, the effector cells transfected with the TCR of the present disclosure have no killing effect on target cells loaded with other short peptides.

### Example 7. Killing function LDH assay for effector cells transfected with TCR of the present disclosure against tumor cell lines.

In this example, a tumor cell line was utilized to retest the killing function and specificity of the cells transfected with the TCR of the present disclosure. The release of LDH is also determined by non-radioactive cytotoxicity assays well known to those skilled in the art. The CD3+ T cells isolated from the blood of healthy volunteers were transfected with CD3+ T cells of the present disclosure and were then used as effector cells, and CD3+ T cells transfected with other TCRs (A6) of the same volunteer were used as the negative control. The numbering of the TCR and the sequences of the alpha and beta variable domains thereof were shown in Table 2. A375-E6 (HPV16 E6 overexpression), and HK-2 were used as HPV16 E6 positive tumor cell lines, and A375 and HCCC9810 as the negative tumor cell lines.

The procedure of the assay was as shown in Example 6, wherein each of the components was added to the ELISPOT plate, wherein the target cells were 3×10⁴ cells/well and the effector cells were 3×10⁴ cells/well (calculated based on the positive rate of transfection). The assay was performed in triplicate.

As shown in FIG.11, the effector cells transfected with the TCR of the present disclosure also show a strong killing effect against HPV16 E6-positive tumor cell lines, while the T cells transfected with other TCR were substantially not effective. Meanwhile, the T cells transfected with the TCR of the present disclosure substantially did not kill the negative tumor cell lines, further showing the good specific killing function of the cells transfected with the TCR of the present disclosure.

### Example 8. Verification of the immune efficacy of effector cells transfected with TCR molecule of the present disclosure against tumor cell lines by IncuCyte

In this example, the effector cells transfected with the TCR of the present disclosure were further verified by those skilled in the art using the IncuCyte assay. IncuCyte is a functional analysis system that can automatically analyze images from real-time microscopic shooting at different time points in an incubator and quantify real-time apoptosis numbers.

The CD3+ T cells isolated from the blood of healthy volunteers were transfected with CD3+ T cells of the present disclosure and were then used as effector cells, and CD3+ T cells empty transfected of the same volunteer were used as control group. The numbering of the TCR and the sequences of the alpha and beta variable domains thereof were shown in Table 2. In this example, the assay was performed with positive target cell CASKI.

On day 1 of the assay, the target cells were digested, centrifuged, resuspended in a complete medium of phenol red-free RPMI1640 plus 10% FBS, seeded evenly in a 96-well plate at 1.5×10⁴ cells/well, and incubated at 37°C in a 5%CO₂ incubator overnight. On day 2, the medium in the 96-well plate was discarded and replaced with medium of phenolic red-free RPMI1640 plus 10% FBS containing a dye caspase 3/7 reagent at a dye concentration of 2 drops/ml. The old culture medium was discarded and replaced with fresh medium of phenol red-free RPMI1640 plus 10% FBS. 1.5^{∗}10⁴ cells/well of the effector cells (calculated based on the positive rate of transfection) were co-incubated with the experimental group in which the target cells had been seeded. The plate was placed in a real-time dynamic living cell imaging analyzer-IncuCyte ZooM special for Incucyte testing and incubated for half an hour. Then, real-time observation was started and photos were taken. The data were processed, analyzed and exported using IncuCyte ZooM 2016A.

As shown in FIG. 12, the cells transfected with the TCR of the present disclosure enabled a significant killer effect in a short period of time against positive tumor cell lines.

All references to the present disclosure are incorporated by reference in this application as if each of them was individually incorporated by reference. It is further to be understood that, after reading the foregoing teachings of the present disclosure, those skilled in the art may make various changes or modifications to the present disclosure and that these equivalents fall within the scope of the claims appended hereto likewise.

## Claims

1. A T cell receptor (TCR) having an activity to bind to TIHDIILECV-HLA A0201 complex, wherein the TCR has an alpha chain variable domain having an amino acid sequence having at least 90% sequence homology to the amino acid sequence as set forth in SEQ ID NO: 1, and a beta chain variable domain having an amino acid sequence having at least 90% sequence homology to the amino acid sequence as set forth in SEQ ID NO: 5.

2. The TCR of claim 1, wherein the TCR is an αβ heterodimeric TCR, preferably the TCR has an alpha chain constant region sequence TRAC^{∗}01 and a beta chain constant region sequence TRBC1^{∗}01 or TRBC2^{∗}01.

3. The TCR of claim 1, wherein the TCR is soluble.

4. The TCR of claim 1, wherein the alpha chain variable domain of the TCR comprises three CDRs, the beta chain variable domain of the TCR comprises three CDRs, and the three CDRs of the beta chain variable domain of the TCR have amino acid sequences of:
β CDR1- MNHNS,
β CDR2- SASEGT, and
β CDR3- ASGPWGSSGNTIY, respectively.

5. The TCR of claim 1, wherein the alpha chain variable domain of the TCR has an amino acid sequence having at least 95% sequence homology to the amino acid sequence as set forth in SEQ ID NO: 1; and the beta chain variable domain of the TCR has an amino acid sequence having at least 95% sequence homology to the amino acid sequence as set forth in SEQ ID NO: 5.

6. The TCR of claim 1, wherein the beta chain variable domain of the TCR has an amino acid sequence as set forth in SEQ ID NO:5.

7. The TCR of claim 1, wherein the alpha chain variable domain of the TCR has an amino acid sequence having at least 95% sequence homology to the amino acid sequence as set forth in SEQ ID NO: 1; and the three CDRs of the beta chain variable domain of the TCR have amino acid sequences of:
β CDR1- MNHNS,
β CDR2- SASEGT, and
β CDR3- ASGPWGSSGNTIY, respectively.

8. The TCR of claim 1, wherein the TCR has CDRs selected from the group consisting of:
| CDR No. | CDR1α | CDR2α | CDR3α | CDR1β | CDR2β | CDR3β |
|---|---|---|---|---|---|---|
| 1 | TRDTTYY | RNSFDEQN | ALRAGANNLF | MNHNS | SASEGT | ASGPWGSSGNTIY |
| 2 | TRDTTYY | RNSFDEQN | ALRAGANLPV | MNHNS | SASEGT | ASGPWGSSGNTIY |
| 3 | TRDTTYY | RNSFDEQN | ALRAGANTPI | MNHNS | SASEGT | ASGPWGSSGNTIY |
| 4 | TRDTTYY | RNSFDEQN | ALRAGAWPMK | MNHNS | SASEGT | ASGPWGSSGNTIY |

9. The TCR of claim 1, wherein the amino acid sequence of the alpha chain variable domain of the TCR is as set forth in any one of SEQ ID NOs: 1 to 4; and the amino acid sequence of the beta chain variable domain of the TCR is as set forth in SEQ ID NO: 5.

10. The TCR of claim 1, wherein the TCR comprises (i) the alpha chain variable domain of the TCR and all or part of an alpha chain constant region of the TCR other than a transmembrane domain and (ii) the beta chain variable domain of the TCR and all or part of a beta chain constant region of the TCR other than a transmembrane domain.

11. The TCR of claim 1, wherein the TCR comprises an alpha chain constant region and a beta chain constant region, and an artificial interchain disulfide bond is comprised between the alpha chain constant region and the beta chain constant region, preferably, a cysteine residue forming the artificial interchain disulfide bond is substituted for one or more sets of sites selected from the group consisting of:
Thr48 of TRAC^{∗}01 exon 1 and Ser57 of TRBC1^{∗}01 or TRBC2^{∗}01 exon 1;
Thr45 of TRAC^{∗}01 exon 1 and Ser77 of TRBC1^{∗}01 or TRBC2^{∗}01 exon 1;
Tyr10 ofTRAC^{∗}01 exon 1 and Ser17 of TRBC1^{∗}01 or TRBC2^{∗}01 exon 1;
Thr45 of TRAC^{∗}01 exon 1 andAsp59 of TRBC1^{∗}01 or TRBC2^{∗}01 exon 1;
Ser15 of TRAC^{∗}01 exon 1 and Glu15 of TRBC1^{∗}01 or TRBC2^{∗}01 exon 1;
Arg53 of TRAC^{∗}01 exon 1 and Ser54 of TRBC1^{∗}01 or TRBC2^{∗}01 exon 1;
Pro89 of TRAC^{∗}01 exon 1 and Ala19 of TRBC1^{∗}01 orTRBC2^{∗}01 exon 1; and
Tyr10 of TRAC^{∗}01 exon 1 and Glu20 of TRBC1^{∗}01 or TRBC2^{∗}01 exon 1.

12. The TCR of claim 1, wherein the TCR is a single-stranded TCR, preferably, the single-stranded TCR is the alpha chain variable domain linked to the beta chain variable domain via a flexible short peptide sequence (linker).

13. The TCR of any one of the preceding claims, wherein the alpha chain and/or the beta chain of the TCR is bound with a conjugate at the C- or N-terminus, preferably, the conjugate is an anti-CD3 antibody.

14. A multivalent TCR complex comprising at least two TCR molecules, wherein at least one of the TCR molecules is a TCR of any one of the preceding claims.

15. A nucleic acid molecule comprising a nucleic acid sequence encoding a TCR of claim 1, or a complementary sequence thereto.

16. A vector comprising a nucleic acid molecule of claim 15.

17. A host cell comprising a vector of claim 16 or having a genome into which an exogenous nucleic acid molecule of claim 15 is integrated.

18. An isolated cell, wherein the cell expresses a TCR of claim 1, preferably, the cell is a T cell.

19. A pharmaceutical composition comprising a TCR of claim 1, a TCR complex of claim 14, or a cell of claim 18, and a pharmaceutically acceptable carrier.

20. A method for treating a disease comprising administering to a subject in need thereof a TCR of claim 1, a TCR complex of claim 14, or a cell of claim 18, and a pharmaceutically acceptable carrier.

21. Use of a TCR of claim 1, a TCR complex of claim 14, or a cell of claim 18 in manufacturing a medicament for treating a tumor, preferably, the tumor is an HPV16 E6-positive tumor.

22. A TCR of claim 1, a TCR complex of claim 14, or a cell of claim 18 for use as a medicament for treating a tumor, preferably, the tumor is an HPV16 E6-positive tumor.

23. A method for preparing a T cell receptor of claim 1, comprising the steps of:
(i) culturing a host cell of claim 17 to express a T cell receptor of claim 1; and
(ii) isolating or purifying the T cell receptor.
